# FASCICULE DE BREVET EUROPEEN

(11) **EP 1 707 562 B1**
(45) Date de publication et mention de la délivrance du brevet: **28.05.2008**
(21) Numéro de dépôt: 06290328.1
(22) Date de dépôt: 28.02.2006
(51) Int. Cl.: C07D 223/16, A61K 31/55

(54) **Forme cristalline gamma du chlorhydrate de l'ivabradine, son procede de preparation, et les compositions pharmaceutique qui la contiennent**
Krystallinform gamma von Ivabradine-hydrochlorid, Verfahren zu dessen Herstellung und diesen enthaltende Pharamazeutische Zusammensetzung
Crystalline form gamma of the chlorhydrate of ivabradine, process for its preparation and pharamcetuical composition containing it

(30) Priorité: 28.02.2005 FR 0501989
(43) Date de publication de la demande: 04.10.2006
(73) Titulaire: Les Laboratoires Servier, 92415 Courbevoie Cedex (FR)
(72) Inventeur: Horvath, Stéphane, 45380 La Chapelle-Saint-Mesmin (FR); Auguste, Marie-Noelle, Orleans (FR); Damien, Gérard, 45130 Meung-Sur-Loire (FR)
(74) Mandataire: Giudicelli, Cathy

(56) Documents cités:
- EP-A- 0 534 859

## Description

La présente invention concerne la nouvelle forme cristalline γ du chlorhydrate de l'ivabradine de formule (I), son procédé de préparation ainsi que les compositions pharmaceutiques qui la contiennent.

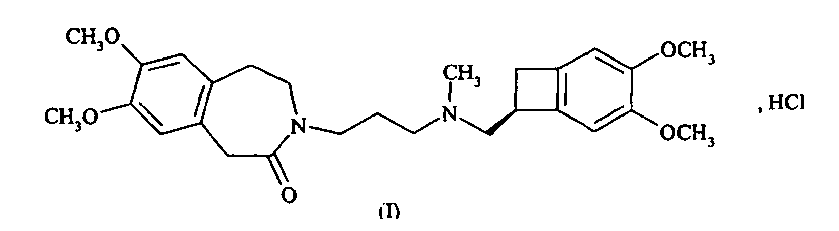

L'ivabradine, ainsi que ses sels d'addition à un acide pharmaceutiquement acceptable, et plus particulièrement son chlorhydrate, ont des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Compte tenu de l'intérêt pharmaceutique de ce composé, il était primordial de l'obtenir avec une excellente pureté. Il était également important de pouvoir le synthétiser selon un procédé facilement transposable à l'échelle industrielle, et notamment sous une forme permettant une filtration et un séchage rapides. Enfin, cette forme devait être parfaitement reproductible, facilement formulée et suffisamment stable pour autoriser son stockage prolongé sans conditions particulières de température, de lumière ou de taux d'oxygène.

Le brevet EP 0534 859 décrit un procédé de synthèse de l'ivabradine et de son chlorhydrate. Cependant, ce document ne précise pas les conditions d'obtention de l'ivabradine sous une forme présentant ces caractéristiques de manière reproductible.

La demanderesse a présentement trouvé qu'un sel particulier de l'ivabradine, le chlorhydrate, pouvait être obtenu sous une forme cristalline bien définie, et présentant des caractéristiques intéressantes de stabilité et de processabilité.

Plus spécifiquement, la présente invention concerne la forme cristalline γ du chlorhydrate de l'ivabradine, caractérisée par le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 4,2 | 1456 | 144 | 0,1004 | 20,762 |
| 2 | 6,9 | 125 | 99 | 0,8029 | 12,880 |
| 3 | 8,4 | 182 | 18 | 0,1004 | 10,503 |
| 4 | 10,7 | 240 | 32 | 0,1338 | 8,249 |
| 5 | 11,3 | 74 | 15 | 0,2007 | 7,858 |
| 6 | 12,0 | 644 | 64 | 0,1004 | 7,392 |
| 7 | 12,5 | 1476 | 219 | 0,1506 | 7,060 |
| 8 | 13,4 | 2691 | 400 | 0,1506 | 6,612 |
| 9 | 14,5 | 541 | 80 | 0,1506 | 6,119 |
| 10 | 14,8 | 104 | 17 | 0,1673 | 5,981 |
| 11 | 15,9 | 815 | 67 | 0,0836 | 5,559 |
| 12 | 16,3 | 501 | 74 | 0,1506 | 5,419 |
| 13 | 17,0 | 1168 | 154 | 0,1338 | 5,210 |
| 14 | 17,9 | 430 | 43 | 0,1004 | 4,962 |
| 15 | 19,0 | 667 | 121 | 0,184 | 4,672 |
| 16 | 19,8 | 527 | 104 | 0,2007 | 4,483 |
| 17 | 20,2 | 726 | 144 | 0,2007 | 4,392 |
| 18 | 20,5 | 282 | 28 | 0,1004 | 4,323 |
| 19 | 21,1 | 2255 | 260 | 0,1171 | 4,208 |
| 20 | 21,4 | 694 | 68 | 0,1004 | 4,147 |
| 21 | 21,6 | 744 | 86 | 0,1171 | 4,111 |
| 22 | 22,3 | 175 | 35 | 0,2007 | 3,987 |
| 23 | 23,5 | 310 | 61 | 0,2007 | 3,784 |
| 24 | 24,2 | 1635 | 270 | 0,1673 | 3,683 |
| 25 | 24,5 | 1335 | 220 | 0,1673 | 3,625 |
| 26 | 24,9 | 523 | 95 | 0,184 | 3,568 |
| 27 | 25,5 | 657 | 130 | 0,2007 | 3,485 |
| 28 | 26,0 | 933 | 154 | 0,1673 | 3,431 |
| 29 | 26,4 | 1549 | 230 | 0,1506 | 3,380 |
| 30 | 26,8 | 419 | 83 | 0,2007 | 3,323 |
| 31 | 27,3 | 350 | 69 | 0,2007 | 3,267 |
| 32 | 28,0 | 1108 | 146 | 0,1338 | 3,186 |
| 33 | 29,1 | 144 | 19 | 0,1338 | 3,066 |

L'invention s'étend également à un procédé de préparation de la forme cristalline γ du chlorhydrate de l'ivabradine, caractérisé en ce que l'on chauffe un mélange de chlorhydrate d'ivabradine et de 2-éthoxyéthanol, un mélange de chlorhydrate d'ivabradine, de 2-éthoxyéthanol et d'eau, ou un mélange de chlorhydrate d'ivabradine, d'éthanol et d'eau jusqu'à dissolution complète, puis on refroidit jusqu'à cristallisation complète et on recueille le produit par filtration.
• Dans le procédé de cristallisation selon l'invention, on peut utiliser le chlorhydrate de l'ivabradine obtenu par n'importe quel procédé, par exemple le chlorhydrate de ivabradine obtenu par le procédé de préparation décrit dans le brevet EP 0534 859.
• La solution peut être avantageusement ensemencée pendant l'étape de refroidissement.

L'invention s'étend aussi aux compositions pharmaceutiques renfermant comme principe actif la forme cristalline γ du chlorhydrate de l'ivabradine avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les exemples suivants illustrent l'invention.

Le spectre de diffraction X sur poudre a été mesuré avec les conditions expérimentales suivantes :
- Diffractomètre PANalytical X'Pert Pro, détecteur X'Celerator, chambre régulée en température,
- Tension 45 KV, intensité 40mA,
- Montage θ-θ,
- Filtre nickel (Kβ),
- Fente de Soller sur le faisceau incident et sur le faisceau diffracté : 0,04 rad,
- Fentes de divergence fixe : angle 1/8°,
- Masque : 10 mm,
- Fente anti-diffusion : 1/4°,
- Mode de mesure : continu de 3° à 30°, avec une incrémentation de 0,017°,
- Temps de mesure par pas : 19,7 s,
- Temps total : 4 min 32 s,
- Vitesse de mesure : 0,108 °/s.
- Température de mesure : ambiante.

### EXEMPLE 1 : Forme cristalline γ du chlorhydrate de l'ivabradine

40 ml de 2-éthoxyéthanol sont préchauffés à 80°C, puis 8,4 g du chlorhydrate de l'ivabradine obtenu selon le procédé décrit dans le brevet EP 0534 859 sont ajoutés par portions sous agitation, et le milieu est chauffé à 80°C jusqu'à dissolution complète. Après retour à température ambiante, la solution est stockée pendant 8 jours, puis les cristaux formés sont recueillis par filtration et rincés par du cyclohexane.

La teneur en eau des cristaux obtenus, déterminée par coulométrie, est de 3,5%, ce qui correspond à un monohydrate.

### Diagramme de diffraction X sur poudre :

Le profil de diffraction des rayons X de la poudre (angles de diffraction) de la forme γ du chlorhydrate de l'ivabradine est donné par les raies significatives rassemblées dans le tableau suivant :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 4,2 | 1456 | 144 | 0,1004 | 20,762 |
| 2 | 6,9 | 125 | 99 | 0,8029 | 12,880 |
| 3 | 8,4 | 182 | 18 | 0,1004 | 10,503 |
| 4 | 10,7 | 240 | 32 | 0,1338 | 8,249 |
| 5 | 11,3 | 74 | 15 | 0,2007 | 7,858 |
| 6 | 12,0 | 644 | 64 | 0,1004 | 7,392 |
| 7 | 12,5 | 1476 | 219 | 0,1506 | 7,060 |
| 8 | 13,4 | 2691 | 400 | 0,1506 | 6,612 |
| 9 | 14,5 | 541 | 80 | 0,1506 | 6,119 |
| 10 | 14,8 | 104 | 17 | 0,1673 | 5,981 |
| 11 | 15,9 | 815 | 67 | 0,0836 | 5,559 |
| 12 | 16,3 | 501 | 74 | 0,1506 | 5,419 |
| 13 | 17,0 | 1168 | 154 | 0,1338 | 5,210 |
| 14 | 17,9 | 430 | 43 | 0,1004 | 4,962 |
| 15 | 19,0 | 667 | 121 | 0,184 | 4,672 |
| 16 | 19,8 | 527 | 104 | 0,2007 | 4,483 |
| 17 | 20,2 | 726 | 144 | 0,2007 | 4,392 |
| 18 | 20,5 | 282 | 28 | 0,1004 | 4,323 |
| 19 | 21,1 | 2255 | 260 | 0,1171 | 4,208 |
| 20 | 21,4 | 694 | 68 | 0,1004 | 4,147 |
| 21 | 21,6 | 744 | 86 | 0,1171 | 4,111 |
| 22 | 22,3 | 175 | 35 | 0,2007 | 3,987 |
| 23 | 23,5 | 310 | 61 | 0,2007 | 3,784 |
| 24 | 24,2 | 1635 | 270 | 0,1673 | 3,683 |
| 25 | 24,5 | 1335 | 220 | 0,1673 | 3,625 |
| 26 | 24,9 | 523 | 95 | 0,184 | 3,568 |
| 27 | 25,5 | 657 | 130 | 0,2007 | 3,485 |
| 28 | 26,0 | 933 | 154 | 0,1673 | 3,431 |
| 29 | 26,4 | 1549 | 230 | 0,1506 | 3,380 |
| 30 | 26,8 | 419 | 83 | 0,2007 | 3,323 |
| 31 | 27,3 | 350 | 69 | 0,2007 | 3,267 |
| 32 | 28,0 | 1108 | 146 | 0,1338 | 3,186 |
| 33 | 29,1 | 144 | 19 | 0,1338 | 3,066 |

### EXEMPLE 2 : Composition pharmaceutique

Formule de préparation pour 1000 comprimés dosés à 5 mg d'ivabradine base:

| | |
|---|---|
| Composé de l'exemple 1 | 5,39 g |
| Amidon de maïs | 20 g |
| Silice colloïdale anhydre | 0,2 g |
| Mannitol | 63,91 g |
| PVP | 10 g |
| Stéarate de magnésium | 0,5 g |

## Revendications

1. Forme cristalline γ du chlorhydrate de l'ivabradine de formule (I) : 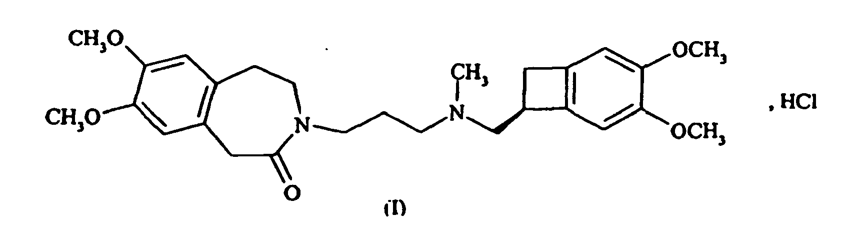 **caractérisée par** le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :
| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 4,2 | 1456 | 144 | 0,1004 | 20,762 |
| 2 | 6,9 | 125 | 99 | 0,8029 | 12,880 |
| 3 | 8,4 | 182 | 18 | 0,1004 | 10,503 |
| 4 | 10,7 | 240 | 32 | 0,1338 | 8,249 |
| 5 | 11,3 | 74 | 15 | 0,2007 | 7,858 |
| 6 | 12,0 | 644 | 64 | 0,1004 | 7,392 |
| 7 | 12,5 | 1476 | 219 | 0,1506 | 7,060 |
| 8 | 13,4 | 2691 | 400 | 0,1506 | 6,612 |
| 9 | 14,5 | 541 | 80 | 0,1506 | 6,119 |
| 10 | 14,8 | 104 | 17 | 0,1673 | 5,981 |
| 11 | 15,9 | 815 | 67 | 0,0836 | 5,559 |
| 12 | 16,3 | 501 | 74 | 0,1506 | 5,419 |
| 13 | 17,0 | 1168 | 154 | 0,1338 | 5,210 |
| 14 | 17,9 | 430 | 43 | 0,1004 | 4,962 |
| 15 | 19,0 | 667 | 121 | 0,184 | 4,672 |
| 16 | 19,8 | 527 | 104 | 0,2007 | 4,483 |
| 17 | 20,2 | 726 | 144 | 0,2007 | 4,392 |
| 18 | 20,5 | 282 | 28 | 0,1004 | 4,323 |
| 19 | 21,1 | 2255 | 260 | 0,1171 | 4,208 |
| 20 | 21,4 | 694 | 68 | 0,1004 | 4,147 |
| 21 | 21,6 | 744 | 86 | 0,1171 | 4,111 |
| 22 | 22,3 | 175 | 35 | 0,2007 | 3,987 |
| 23 | 23,5 | 310 | 61 | 0,2007 | 3,784 |
| 24 | 24,2 | 1635 | 270 | 0,1673 | 3,683 |
| 25 | 24,5 | 1335 | 220 | 0,1673 | 3,625 |
| 26 | 24,9 | 523 | 95 | 0,184 | 3,568 |
| 27 | 25,5 | 657 | 130 | 0,2007 | 3,485 |
| 28 | 26,0 | 933 | 154 | 0,1673 | 3,431 |
| 29 | 26,4 | 1549 | 230 | 0,1506 | 3,380 |
| 30 | 26,8 | 419 | 83 | 0,2007 | 3,323 |
| 31 | 27,3 | 350 | 69 | 0,2007 | 3,267 |
| 32 | 28,0 | 1108 | 146 | 0,1338 | 3,186 |
| 33 | 29,1 | 144 | 19 | 0,1338 | 3,066 |

2. Procédé de préparation de la forme cristalline γ du chlorhydrate de l'ivabradine selon la revendication 1, **caractérisé en ce que** l'on chauffe un mélange de chlorhydrate d'ivabradine et de 2-éthoxyéthanol, un mélange de chlorhydrate d'ivabradine, de 2-éthoxyéthanol et d'eau, ou un mélange de chlorhydrate d'ivabradine, d'éthanol et d'eau jusqu'à dissolution complète, puis on refroidit jusqu'à cristallisation complète et on recueille le produit par filtration.

3. Procédé selon la revendication 2, **caractérisé en ce que** la solution du chlorhydrate de l'ivabradine est ensemencée pendant l'étape de refroidissement.

4. Composition pharmaceutique contenant comme principe actif la forme cristalline γ du chlorhydrate de l'ivabradine selon la revendication 1, en combinaison avec un ou plusieurs véhicules inertes, non toxiques et pharmaceutiquement acceptables.

5. Utilisation de la forme cristalline γ du chlorhydrate de l'ivabradine selon la revendication 1, pour la fabrication de médicaments utiles comme bradycardisants.

6. Utilisation de la forme cristalline γ du chlorhydrate de l'ivabradine selon la revendication 1, pour la fabrication de médicaments utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

## Claims

1. γ-Crystalline form of ivabradine hydrochloride of formula (I) : 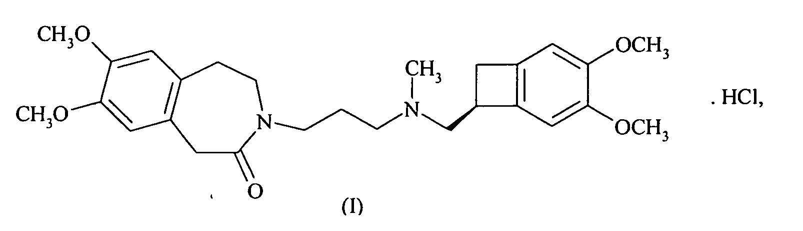 **characterised by** the following powder X-ray diffraction diagram measured using a PANalytical X'Pert Pro diffractometer together with an X'Celerator detector and expressed in terms of line position (Bragg's angle 2 theta, expressed in degrees), line height (expressed in counts), line area (expressed in counts x degrees), line width at half-height ("FWHM", expressed in degrees) and interplanar distance d (expressed in Å):
| **Line no.** | **Angle 2 theta (degrees)** | **Height (counts)** | **Area (counts x degrees)** | **FWHM (degrees)** | **Interplanar distance (Å)** |
|---|---|---|---|---|---|
| 1 | 4.2 | 1456 | 144 | 0.1004 | 20.762 |
| 2 | 6.9 | 125 | 99 | 0.8029 | 12.880 |
| 3 | 8.4 | 182 | 18 | 0.1004 | 10.503 |
| 4 | 10.7 | 240 | 32 | 0.1338 | 8.249 |
| 5 | 11.3 | 74 | 15 | 0.2007 | 7.858 |
| 6 | 12.0 | 644 | 64 | 0.1004 | 7.392 |
| 7 | 12.5 | 1476 | 219 | 0.1506 | 7.060 |
| 8 | 13.4 | 2691 | 400 | 0.1506 | 6.612 |
| 9 | 14.5 | 541 | 80 | 0.1506 | 6.119 |
| 10 | 14.8 | 104 | 17 | 0.1673 | 5.981 |
| 11 | 15.9 | 815 | 67 | 0.0836 | 5.559 |
| 12 | 16.3 | 501 | 74 | 0.1506 | 5.419 |
| 13 | 17.0 | 1168 | 154 | 0.1338 | 5.210 |
| 14 | 17.9 | 430 | 43 | 0.1004 | 4.962 |
| 15 | 19.0 | 667 | 121 | 0.184 | 4.672 |
| 16 | 19.8 | 527 | 104 | 0.2007 | 4.483 |
| 17 | 20.2 | 726 | 144 | 0.2007 | 4.392 |
| 18 | 20.5 | 282 | 28 | 0.1004 | 4.323 |
| 19 | 21.1 | 2255 | 260 | 0.1171 | 4.208 |
| 20 | 21.4 | 694 | 68 | 0.1004 | 4.147 |
| 21 | 21.6 | 744 | 86 | 0.1171 | 4.111 |
| 22 | 22.3 | 175 | 35 | 0.2007 | 3.987 |
| 23 | 23.5 | 310 | 61 | 0.2007 | 3.784 |
| 24 | 24.2 | 1635 | 270 | 0.1673 | 3.683 |
| 25 | 24.5 | 1335 | 220 | 0.1673 | 3.625 |
| 26 | 24.9 | 523 | 95 | 0.184 | 3.568 |
| 27 | 25.5 | 657 | 130 | 0.2007 | 3.485 |
| 28 | 26.0 | 933 | 154 | 0.1673 | 3.431 |
| 29 | 26.4 | 1549 | 230 | 0.1506 | 3.380 |
| 30 | 26.8 | 419 | 83 | 0.2007 | 3.323 |
| 31 | 27.3 | 350 | 69 | 0.2007 | 3.267 |
| 32 | 28.0 | 1108 | 146 | 0.1338 | 3.186 |
| 33 | 29.1 | 144 | 19 | 0.1338 | 3.066 |

2. Process for the preparation of the γ-crystalline form of ivabradine hydrochloride according to claim 1, **characterised in that** a mixture of ivabradine hydrochloride and 2-ethoxyethanol, a mixture of ivabradine hydrochloride, 2-ethoxyethanol and water, or a mixture of ivabradine hydrochloride, ethanol and water is heated until dissolution is complete and is then cooled until crystallisation is complete, and the product is collected by filtration.

3. Process according to claim 2, **characterised in that** the solution of ivabradine hydrochloride is seeded during the cooling step.

4. Pharmaceutical composition comprising as active ingredient the γ-crystalline form of ivabradine hydrochloride according to claim 1, in combination with one or more pharmaceutically acceptable, inert, non-toxic carriers.

5. Use of the γ-crystalline form of ivabradine hydrochloride according to claim 1 in the manufacture of medicaments which are of use as bradycardics.

6. Use of the γ-crystalline form of ivabradine hydrochloride according to claim 1 in the manufacture of medicaments which are of use in the treatment or prevention of various clinical situations of myocardial ischaemia such as angina pectoris, myocardial infarct and associated rhythm disturbances, and also in various pathologies involving rhythm disturbances, especially supraventricular rhythm disturbances, and in heart failure.

## Patentansprüche

1. γ-Kristallform des Hydrochlorids von Ivabradin der Formel (I): 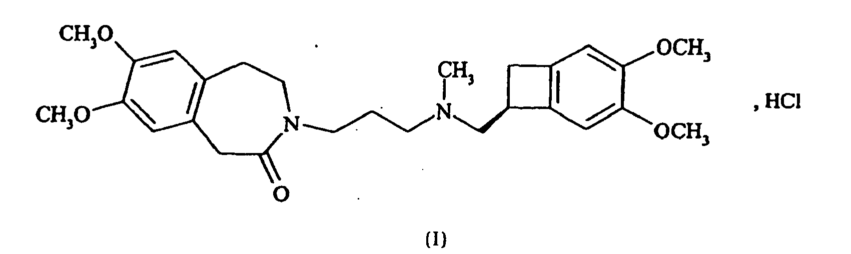 **gekennzeichnet durch** das folgende Pulverröntgenbeugungsdiagramm, welches mit einem Diffraktometer PANalytical X'Pert Pro mit einem Detektor X'Celerator gemessen worden ist und angegeben ist mit der Position des Peaks (Bragg 2 Theta-Winkel, angegeben in Grad), Höhe des Peaks (angegeben in Zählimpulsen), Fläche des Peaks (angegeben in Zählimpulsen x Grad), Halbwertsbreite der Peaks ("FWHM", angegeben in Grad) und den Gitterabstand d (angegeben in Å):
| Peak Nr. | 2 Theta-Winkel (Grad) | Höhe (Zähl-impulse) | Fläche (Zählimpulse × Grad) | FWHM (Grad) | Gitter-abstand (Å) |
|---|---|---|---|---|---|
| 1 | 4,2 | 1456 | 144 | 0,1004 | 20,762 |
| 2 | 6,9 | 125 | 99 | 0.8029 | 12,880 |
| 3 | 8,4 | 182 | 18 | 0,1004 | 10,503 |
| 4 | 10,7 | 240 | 32 | 0,1338 | 8,249 |
| 5 | 11,3 | 74 | 15 | 0,2007 | 7,858 |
| 6 | 12,0 | 644 | 64 | 0,1004 | 7,392 |
| 7 | 12,5 | 1476 | 219 | 0,1506 | 7,060 |
| 8 | 13,4 | 2691 | 400 | 0,1506 | 6,612 |
| 9 | 14,5 | 541 | 80 | 0,1506 | 6,119 |
| 10 | 14,8 | 104 | 17 | 0,1673 | 5,981 |
| 11 | 15,9 | 815 | 67 | 0,0836 | 5,559 |
| 12 | 16,3 | 501 | 74 | 0,1506 | 5,419 |
| 13 | 17,0 | 1168 | 154 | 0,1338 | 5,210 |
| 14 | 17,9 | 430 | 43 | 0,1004 | 4,962 |
| 15 | 19,0 | 667 | 121 | 0,184 | 4,672 |
| 16 | 19,8 | 527 | 104 | 0,2007 | 4,483 |
| 17 | 20,2 | 726 | 144 | 0,2007 | 4,392 |
| 18 | 20,5 | 282 | 28 | 0,1004 | 4,323 |
| 19 | 21,1 | 2255 | 260 | 0,1171 | 4,208 |
| 20 | 21,4 | 694 | 68 | 0,1004 | 4,147 |
| 21 | 21,6 | 744 | 86 | 0,1171 | 4.111 |
| 22 | 22,3 | 175 | 35 | 0,2007 | 3.987 |
| 23 | 23,5 | 310 | 61 | 0,2007 | 3,784 |
| 24 | 24,2 | 1635 | 270 | 0,1673 | 3,683 |
| 25 | 24,5 | 1335 | 220 | 0,1673 | 3,625 |
| 26 | 24,9 | 523 | 95 | 0,184 | 3,568 |
| 27 | 25,5 | 657 | 130 | 0,2007 | 3,485 |
| 28 | 26,0 | 933 | 154 | 0,1673 | 3,431 |
| 29 | 26,4 | 1549 | 230 | 0.1506 | 3,380 |
| 30 | 26,8 | 419 | 83 | 0,2007 | 3,323 |
| 31 | 27,3 | 350 | 69 | 0,2007 | 3,267 |
| 32 | 28,0 | 1108 | 146 | 0,1338 | 3,186 |
| 33 | 29,1 | 144 | 19 | 0,1338 | 3,006 |

2. Verfahren zur Herstellung der γ-Kristallform des Hydrochlorids von Ivabradin nach Anspruch 1, **dadurch gekennzeichnet, dass** man eine Mischung aus Ivabradin-Hydrochlorid und 2-Ethoxyethanol, eine Mischung aus Ivabradin-Hydrochlorid, 2-Ethoxyethanol und Wasser oder eine Mischung aus Ivabradin-Hydrochlorid, Ethanol und Wasser bis zur vollständigen Auflösung erhitzt, dann progressiv bis zur vollständigen Kristallisation abkühlt und das Produkt durch Filtration gewinnt.

3. Verfahren nach Anspruch 2, **dadurch gekennzeichnet, dass** die Lösung von Ivabradin-Hydrochlorid während der Stufe des Abkühlens angeimpft wird.

4. Pharmazeutische Zubereitung enthaltend als Wirkstoff die γ-Kristallform des Hydrochlorids von Ivabradin nach Anspruch 1 in Kombination mit einem oder mehreren inerten, nichttoxischen und pharmazeutisch annehmbaren Hilfsstoffen.

5. Verwendung der γ-Kristallform des Hydrochlorids von Ivabradin nach Anspruch 1 für die Herstellung von Arzneimitteln, die als bradykardisierende Mittel geeignet sind.

6. Verwendung der γ-Kristallform des Hydrochlorids von Ivabradin nach Anspruch 1 für die Herstellung von Arzneimitteln, die nützlich sind bei der Behandlung oder der Vorbeugung von verschiedenen klinischen Zuständen der Myokardischämie, wie Angina pectoris, Myokardinfarkt und damit verknüpften Rhythmusstörungen, sowie von verschiedenen Erkrankungen, bei denen Rhythmusstörungen auftreten, insbesondere supra-ventrikuläre, und bei der Behandlung von Herzinsuffizienz.
